# EUROPEAN PATENT APPLICATION

(11) **EP 4 386 385 A1**
(43) Date of publication of application: **19.06.2024**
(21) Application number: 22855879.7
(22) Date of filing: 09.08.2022
(51) Int. Cl.: G01N 33/531, G01N 33/543

(54) **IMMUNOASSAY METHOD, SPECIMEN DILUENT, AND IMMUNOCHROMATOGRAPHY KIT**

(30) Priority: 12.08.2021 JP 2021131690
(71) Applicant: Sekisui Medical Co., Ltd., Tokyo 103-0027 (JP)
(72) Inventor: YAJI, Shohei, Tokyo 103-0027 (JP); ITO, Shizuka, Tokyo 103-0027 (JP); OKUYAMA, Shinya, Tokyo 103-0027 (JP); OCHIAI, Yasushi, Tokyo 103-0027 (JP); KOHNO, Keigo, Tokyo 103-0027 (JP); WATANABE, Keisuke, Tokyo 103-0027 (JP); HEWSON, Christopher Kenta, Tokyo 103-0027 (JP); SAKAI, Shun, Tokyo 103-0027 (JP)
(74) Representative: Ter Meer Steinmeister & Partner
(86) International application number: PCT/JP2022/030362
(87) International publication number: WO 2023/017817

(57) **Abstract**

The present invention provides an immunoassay method for detecting a protein with an isoelectric point of 9.5 or higher, comprising a step of bringing a sample containing the protein with an isoelectric point of 9.5 or higher into contact with a glass material in the presence of a cationic substance, and an immunochromatography kit for detecting a protein with an isoelectric point of 9.5 or higher, comprising a specimen diluent and an immunochromatography test strip.

## Description

### [Technical Field]

The present invention relates to an immunoassay method, a specimen diluent, and an immunochromatography kit.

Priority is claimed on Japanese Patent Application No. 2021-131690, filed August 12, 2021, the contents of which are incorporated herein by reference.

### [Background Art]

An immunoassay method is a method of measuring the level of a substance (amount, concentration, presence or absence of a substance) contained in a sample using a reaction between an antigen and an antibody. Examples of immunoassay methods include ELISA and immunochromatography.

Immunoassay methods are used to detect foreign substances or metabolites that exist in extremely small amounts in the body. Therefore, immunoassay methods need to be able to detect these foreign substances or metabolites with high sensitivity.

In Patent Literature 1, influenza virus is detected using an immunoassay method.

### [Citation List]

### [Patent Literature]

Patent Document 1: Japanese Unexamined Patent Application Publication No. 2012-233928

### [Summary of Invention]

### [Technical Problem]

An object of the present invention is to provide an immunoassay method and an immunochromatography kit that can detect proteins with an isoelectric point of 9.5 or higher with high sensitivity.

### [Solution to Problem]

The present invention includes the following embodiments.
[1] An immunoassay method for detecting a protein with an isoelectric point of 9.5 or higher, comprising:
   a step of bringing a sample containing the protein with an isoelectric point of 9.5 or higher into contact with a glass material in the presence of a cationic substance.
[2] The immunoassay method according to [1], wherein the sample is contacted with the cationic substance, followed by bringing the protein with an isoelectric point of 9.5 or higher into contact with the glass material.
[3] The immunoassay method according to [1] or [2], wherein the glass material is a specimen filtration filter containing glass fibers.
[4] The immunoassay method according to [1] or [2], which further comprises, prior to the step of bringing the sample into contact with the glass material,
   a step of contacting a sample containing the protein with an isoelectric point of 9.5 or more with a specimen diluent,
   wherein the cationic substance is contained in the specimen diluent.
[5] The immunoassay method according to any one of [1] to [4], wherein the cationic substance is at least one selected from the group consisting of a metal salt, a cationic polymer, and a cationic surfactant.
[6] The immunoassay method according to any one of [1] to [4], wherein the cationic substance is a mixture of a metal salt and a cationic polymer.
[7] The immunoassay method according to any one of [1] to [6], which is immunochromatography.
[8] The immunoassay method according to [7], wherein the glass material is at least one selected from the group consisting of a sample pad, a third pad, and a conjugate pad of an immunochromatography test strip.
[9] The immunoassay method according to any one of [1] to [8], which further comprises:
   a step of contacting the protein with an isoelectric point of 9.5 or higher with a first binding partner to which a labeling substance is directly or indirectly bound, thereby forming a first complex;
   a step of contacting the first complex with a second binding partner to form a second complex; and
   a step of detecting a signal derived from the labeling substance.
[10] The immunoassay method according to any one of [1] to [9], wherein the protein with an isoelectric point of 9.5 or higher is derived from a respiratory infection virus.
[11] The immunoassay method according to [10], wherein the respiratory infection virus is a coronavirus.
[12] The immunoassay method according to [11], wherein the coronavirus is SARS-CoV-2.
[13] An immunochromatography kit for detecting a protein with an isoelectric point of 9.5 or higher, comprising a specimen diluent and an immunochromatography test strip, which further comprises:
   a cationic substance to be contacted with a sample containing the protein with an isoelectric point of 9.5 or higher; and a glass material to be contacted with the sample containing the protein having with an isoelectric point of 9.5 or higher.
[14] The immunochromatography kit according to [13], which has a specimen filtration filter, wherein the specimen filtration filter comprises the glass material.
[15] The immunochromatography kit according to [13], which comprises at least one member selected from the group consisting of a sample pad in the immunochromatography test strip, a third pad in the immunochromatography test strip, and a conjugate pad in the immunochromatography test strip, wherein the at least one member comprises the glass material.
[16] The immunochromatography kit according to any one of [13] to [15], wherein the cationic substance is contained in the specimen diluent.
[17] The immunochromatography kit according to any one of [13] to [16], wherein the cationic substance is at least one selected from the group consisting of a metal salt, a cationic polymer, and a cationic surfactant.
[18] The immunochromatography kit according to any one of [13] to [17], wherein the cationic substance is a mixture of a metal salt and a cationic polymer.
[19] The immunochromatography kit according to any one of [13] to [18], wherein the protein with an isoelectric point of 9.5 or higher is derived from a respiratory infection virus.
[20] The immunochromatography kit according to [19], wherein the respiratory infection virus is a coronavirus.
[21] The immunochromatography kit according to [20], wherein the coronavirus is SARS-CoV-2.
[22] A specimen diluent comprising a cationic substance for use in the immunoassay method of any one of [1] to [12].
[23] A filtration method for a sample containing a protein with an isoelectric point of 9.5 or higher, the method comprising:
   a step of contacting a sample containing the protein with an isoelectric point of 9.5 or more with a specimen diluent, and
   a step of filtering the sample diluted with the specimen diluent using a specimen filtration filter containing a glass material,
   wherein the specimen diluent comprises a cationic substance.
[24] An adsorption suppression method for suppressing adsorption of a protein with an isoelectric point of 9.5 or higher to a glass material in an immunoassay method for detecting a protein with an isoelectric point of 9.5 or higher, the method comprising:
   a step of bringing a sample containing the protein with an isoelectric point of 9.5 or higher into contact with a glass material in the presence of a cationic substance.
[25] The immunochromatography kit according to [13], which comprises at least one member selected from the group consisting of a specimen filtration filter, a sample pad in the immunochromatography test strip, a third pad in the immunochromatography test strip, and a conjugate pad in the immunochromatography test strip, wherein the at least one member comprises the glass material.
[26] The immunochromatography kit according to [25], wherein the cationic substance is contained in the specimen diluent.
[27] The immunochromatography kit according to [25] or [26], wherein the cationic substance is at least one selected from the group consisting of a metal salt, a cationic polymer, and a cationic surfactant.
[28] The immunochromatography kit according to any one of [25] to [27], wherein the cationic substance is a mixture of a metal salt and a cationic polymer.
[29] The immunochromatography kit according to any one of [25] to [28], wherein the protein with an isoelectric point of 9.5 or higher is derived from a respiratory infection virus.
[30] The immunochromatography kit according to [29], wherein the respiratory infection virus is a coronavirus.
[31] The immunochromatography kit according to [30], wherein the coronavirus is SARS-CoV-2.

The present invention also includes the following embodiments.
[32] The immunoassay method according to [2], wherein the step of contacting the sample with the cationic substance, followed by bringing the protein with an isoelectric point of 9.5 or higher into contact with a glass material, is
   a step of filtering the sample diluted with a specimen diluent containing the cationic substance, using a specimen filtration filter containing a glass material.
[33] The immunoassay method according to [2], wherein the step of contacting the sample with the cationic substance, followed by bringing the protein with an isoelectric point of 9.5 or higher into contact with a glass material, is
   a step of bringing the sample diluted with a specimen diluent into contact with at least one selected from the group consisting of a sample pad, a third pad, and a conjugate pad of an immunochromatography test strip, each containing glass fibers.
[34] The immunoassay method according to any one of [6] to [12], [32], and [33], wherein the metal salt and the cationic polymer are sodium chloride and a cationic polymer having 2-methacryloyloxyethyl phosphorylcholine as constituent units.
[35] The immunoassay method according to any one of [5] to [12] and [32] to [34], wherein a concentration of the cationic substance when the sample containing the protein with an isoelectric point of 9.5 or higher is brought into contact with the glass material is 0.1 to 1,000 mM for the metal salt, and 0.0001 to 20 % by mass for the cationic polymer or the cationic surfactant.
[36] The immunoassay method according to any one of [1] to [12] and [32] to [35], wherein the sample is a respiratory secretion.

The present invention also includes the following embodiments.
[37] The immunochromatography kit according to any one of [18] to [21] and [28] to [31], wherein the metal salt and the cationic polymer are sodium chloride and a cationic polymer having 2-methacryloyloxyethyl phosphorylcholine as constituent units.
[38] The immunochromatography kit according to any one of [17] to [21], [27] to [31] and [37], wherein a concentration of the cationic substance in terms of a specimen diluent when the sample containing the protein with an isoelectric point of 9.5 or higher is brought into contact with the glass material is 0.1 to 1,000 mM for the metal salt, and 0.0001 to 20 % by mass for the cationic polymer or the cationic surfactant.
[39] The immunochromatography kit according to any one of [17] to [21], [27] to [31], [37] and [38], wherein the sample is a respiratory secretion.

The present invention also includes the following embodiments.
[40] The filtration method according to [23], wherein the cationic substance is at least one selected from the group consisting of a metal salt, a cationic polymer, and a cationic surfactant.
[41] The filtration method according to [23] or [40], wherein the cationic substance is a mixture of a metal salt and a cationic polymer.
[42] The filtration method according to any one of [30], [41] and [42], wherein the protein with an isoelectric point of 9.5 or higher is derived from a respiratory infection virus.
[43] The filtration method according to [42], wherein the respiratory infection virus is a coronavirus.
[44] The filtration method according to [43], wherein the coronavirus is SARS-CoV-2.
[45] The filtration method according to any one of [41] to [33], wherein the metal salt and the cationic polymer are sodium chloride and a cationic polymer having 2-methacryloyloxyethyl phosphorylcholine as constituent units.
[46] The filtration method according to any one of [40] to [45], wherein a concentration of the cationic substance, based on the specimen diluent, is 0.1 to 1,000 mM for the metal salt, and 0.0001 to 20 % by mass for the cationic polymer or the cationic surfactant.
[47] The filtration method according to any one of [23] and [40] to [46], wherein the sample is a respiratory secretion.

The present invention also includes the following embodiments.
[48] The adsorption suppression method according to [24], wherein the sample containing the protein with an isoelectric point of 9.5 or higher is contacted with the cationic substance, followed by bringing the protein into contact with the glass material.
[49] The adsorption suppression method according to [24] or [48], wherein the glass material is a specimen filtration filter containing glass fibers.
[50] The adsorption suppression method according to any one of [24], [48], and [49], which further comprises, prior to the step of contacting,
   a step of contacting a sample containing the protein with an isoelectric point of 9.5 or more with a specimen diluent,
   wherein the cationic substance is contained in the specimen diluent.
[51] The adsorption suppression method according to any one of [24] and [48] to [50], wherein the cationic substance is at least one selected from the group consisting of a metal salt, a cationic polymer, and a cationic surfactant.
[52] The adsorption suppression method according to any one of [24] and [48] to [51], wherein the cationic substance is a mixture of a metal salt and a cationic polymer.
[53] The adsorption suppression method according to any one of [24] and [48] to [52], which is immunochromatography.
[54] The adsorption suppression method according to any one of [24] and [48] to [53], wherein the glass material is at least one selected from the group consisting of a sample pad, a third pad, and a conjugate pad for immunochromatography.
[55] The adsorption suppression method according to any one of [24] and [48] to [54], which further comprises:
   a step of contacting the protein with an isoelectric point of 9.5 or higher with a first binding partner to which a labeling substance is directly or indirectly bound, thereby forming a first complex;
   a step of contacting the first complex with a second binding partner to form a second complex; and
   a step of detecting a signal derived from the labeling substance.
[56] The adsorption suppression method according to any one of [24] and [48] to [55], wherein the protein with an isoelectric point of 9.5 or higher is derived from a respiratory infection virus.
[57] The adsorption suppression method according to [56], wherein the respiratory infection virus is a coronavirus.
[58] The adsorption suppression method according to [57], wherein the coronavirus is SARS-CoV-2.
[59] The adsorption suppression method according to any one of [52] to [58], wherein the metal salt and the cationic polymer are sodium chloride and a cationic polymer having 2-methacryloyloxyethyl phosphorylcholine as constituent units.
[60] The adsorption suppression method according to any one of [51] to [59], wherein a concentration of the cationic substance when the sample containing the protein with an isoelectric point of 9.5 or higher is brought into contact with the glass material is 0.1 to 1,000 mM for the metal salt, and 0.0001 to 20 % by mass for the cationic polymer or the cationic surfactant.
[61] The adsorption suppression method according to any one of [24] and [48] to [60], wherein the sample is a respiratory secretion.

### [Advantageous Effects of Invention]

The present invention can provide an immunoassay method and an immunochromatography kit that can detect proteins with an isoelectric point of 9.5 or higher with high sensitivity.

### [Brief Description of Drawings]

FIG. 1 is a diagram illustrating a mechanism that is assumed to be ineffective in conventional techniques.
FIG. 2 is a diagram illustrating a mechanism that is assumed to be effective in the present invention.
FIG. 3 is a schematic diagram showing the configuration of an immunochromatography test strip used in the experimental examples.
FIG. 4 is a schematic diagram showing an embodiment of an immunochromatography test strip including a sample pad, a conjugate pad, and a third pad.

### [Description of Embodiments]

There are many techniques using filters or porous membranes for specimen filtration to remove insoluble substances derived from biological samples, which affect immunoassays. In Patent Literature 1 as well, a sample is filtered using a specimen filtration filter before performing the immunoassay method. The present inventors found that when a protein with an isoelectric point of 9.5 or higher is allowed to contact a glass material such as a filter or porous membrane for specimen filtration, the protein adsorbs to the glass material and the measurement sensitivity decreases (Analysis Examples 1 to 3). Heretofore, no discussion has been made about decrease in measurement sensitivity caused by adsorption of a detection target substance to a specimen filtration filter or the like, and how to suppress such a decrease in measurement sensitivity.

The present inventors have made further studies and found that by bringing a protein with an isoelectric point of 9.5 or higher into contact with a glass material in the presence of a cationic substance, it becomes possible to prevent adsorption of a protein with an isoelectric point of 9.5 or higher to glass materials. Thus, the present inventors have found that the above-mentioned problem can be solved, and completed the present invention.

Hereinbelow, the descriptions are given separately for various aspects of the invention, but the descriptions, definitions of terms, and embodiments described for a particular aspect are also applicable to the other aspects.

"RapidTesta", "RapidTesta FLU/NEXT", "FUL/NEXT", and "RapidTesta RSV-adeno/NEXT" are trademarks or registered trademarks owned by Sekisui Medical Co., Ltd. in Japan and other countries.

"Lipidure" is a trademark or registered trademark owned by NOF Corporation in Japan and other countries.

"Quartamin" is a trademark or registered trademark owned by Kao Corporation in Japan and other countries.

"Promois" is a trademark or registered trademark owned by Seiwa Kasei Co., Ltd. in Japan and other countries.

"Biacore" is a trademark or registered trademark owned by Cytiva Sweden Aktiebolag in Japan and other countries.

### 1. Immunoassay method for detecting a protein with an isoelectric point of 9.5 or higher

### (Protein with an isoelectric point of 9.5 or higher)

In the immunoassay method of the present invention, the detection target substance is a protein with an isoelectric point of 9.5 or higher. The protein with an isoelectric point of 9.5 or higher is not particularly limited as long as it can be detected using an antigen-antibody reaction, and examples thereof include exogenous substances (viruses, bacteria, administered drugs, etc.), proteins derived from exogenous substances (metabolites of exogenous substances, secretions by bacteria, etc.), and endogenous substances (antibodies, hormones, etc. produced within the body). The protein with an isoelectric point of 9.5 or higher also encompasses protein fragments, i.e., peptide fragments with an isoelectric point of 9.5 or higher.

The detection target substance may be a protein with an isoelectric point of 9.5 or higher, 9.6 or higher, 9.7 or higher, 9.8 or higher, 9.9 or higher, or 10.0 or higher.

Hereinbelow, a protein with an isoelectric point of 9.5 or higher may be simply referred to as a detection target substance.

The detection target substance with an isoelectric point of 9.5 or higher in the immunoassay method of the present invention is preferably a protein derived from a virus, more preferably a protein derived from a respiratory infection virus, even more preferably a protein derived from a coronavirus such as SARS-CoV, MERS-CoV or SARS-CoV-2, most preferably a protein derived from SARS-CoV-2. SARS-CoV-2 is a virus that causes the coronavirus disease 2019 (COVID-19). In the present specification, a protein with an isoelectric point of 9.5 or higher being "derived from a respiratory infection virus" means that the "protein with an isoelectric point of 9.5 or higher" encompasses not only the respiratory infection virus itself, but also parts of the protein (peptide fragments) that constitute a respiratory infection virus.

### (Sample)

In the immunoassay method of the present invention, examples of samples that may contain the detection target substance mainly include a biological sample derived from an organism, an extract obtained by extracting the detection target substance from a biological sample, and the like. Further examples of usable samples that may contain the detection target substance include food samples such as liquid beverages, semi-solid foods, and solid foods, samples from natural environments such as soil, rivers, and seawater, and wipe samples from factory production lines or clean rooms.

In the immunoassay method of the present invention, a biological sample is preferable as the sample that may contain the detection target substance. More specific examples of biological samples include blood, serum, plasma, urine, tears, ear discharge, prostatic fluid, and respiratory secretions. Respiratory secretions are more preferred. In the context of the present specification, the term "respiratory secretion" means bodily fluids secreted in or on the tissues of the respiratory organs. Examples of respiratory secretions include bodily fluids secreted in the nostrils, nasal cavities, pharynx, nasopharynx, and oral cavity, and particularly preferred examples include nasopharyngeal swabs, nasal swabs, saliva, and sputum. In the context of the present specification, "respiratory organs" is a generic term for organs related to respiration, and covers organs ranging from the nasal vestibule to the alveoli (lungs) via the nasal cavity, pharynx, larynx, trachea, bronchi, and bronchioles.

Examples of subjects from which the biological sample is to be collected include humans or animals (e.g., monkeys, dogs, and cats), of which humans are preferable. The biological sample may be a biological sample as it is taken from a subject, or may be a sample obtained by subjecting a collected biological sample to treatments such as dilution and concentration that are usually performed. The person who collects and prepares a biological sample used in the present invention may or may not be identical to the person who performs the immunoassay method of the present invention. Further, the biol ogical sample used in the immunoassay method of the present invention may be one collected or prepared during implementation of the immunoassay method of the present invention, or one previously collected or prepared and stored.

When the biological sample contains a protein derived from SARS-CoV-2 or its peptide fragments as a detection target substance, its concentration is 0.1 to 100,000 TCID₅₀/mL, 1 to 50,000 TCID₅₀/mL, or 10 to 10,000 TCID₅₀/mL.

### (Glass material)

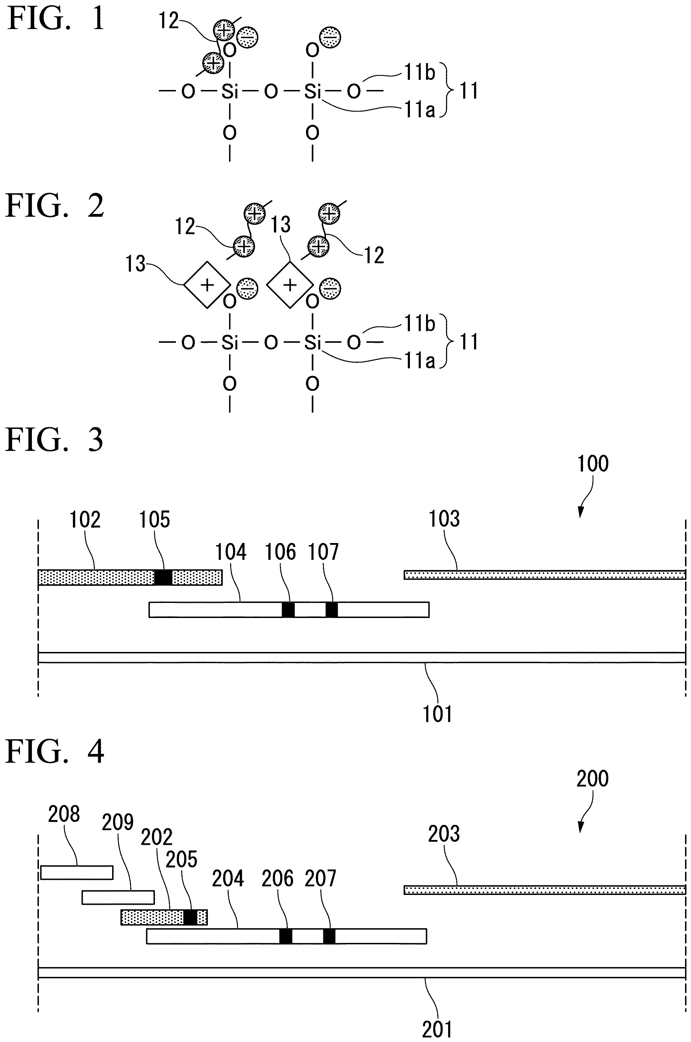

In the present specification, the term "glass material" means an instrument having a glass material in its portion that comes into contact with a protein having an isoelectric point of 9.5 or higher. The term "glass" means glass having silicon dioxide (SiO₂) as a constituent component. In the glass 11 having silicon dioxide (SiO₂) as a constituent component, a network structure based on SiO₄ tetrahedrons with silicon atoms 11a and oxygen atoms 11b positioned at vertices is formed at a side of temperature lower than the glass transition point (FIG. 1 and FIG. 2).

In the conventional techniques, the oxygen atoms 11b bonded to the silicon atoms 11a in the above-mentioned network structure of the glass 11 are negatively charged. It is assumed that the protein 12 having an isoelectric point of 9.5 or higher is adsorbed to this negatively charged oxygen atom 11b, resulting in a decrease in measurement sensitivity (FIG. 1).

In the present invention, it is presumed that by bringing a protein 12 with an isoelectric point of 9.5 or higher into contact with the glass 11 (glass material) in the presence of a cationic substance 13, it becomes possible to suppress adsorption of the protein 12 with an isoelectric point of 9.5 or higher to the negatively charged oxygen atoms 11b of the glass 11.

The above mechanism is hypothetical and does not limit the scope of the present invention in any way.

Examples of glass materials include fiberglass instruments, glass plates, glass tubes, glass beads, and other glass materials.

Fiberglass instruments are instruments that include glass fibers. Examples of fiberglass instruments include a sample pad, a third pad, and a conjugate pad for immunochromatography. A third pad means any pad to be positioned between a sample supply part and an insoluble membrane carrier, which is different from the sample pad and the conjugate pad. Further examples of fiberglass instruments include filters that include glass fibers, such as specimen filtration filters including glass fibers.

Examples of glass plates include a glass-made 96-well immunoplate.

Examples of other glass materials include slide glasses.

### (Cationic substance)

In the present specification, the term "cationic substance" means a substance that has a cationic functional group and becomes positively charged when it comes into contact with water. When the cationic substance has both a cationic functional group and an anionic functional group, it is sufficient that the entire molecule is positively charged.

Examples of cationic functional groups include amino groups such as a primary amino group, a secondary amino group, and a tertiary amino group; onium base groups such as a quaternary ammonium group, and a phosphonium group; amino acid residues such as an arginyl group, a lysyl group, a histidyl group, and a guanidyl group; and heterocyclic groups such as an imidazole group.

The cationic substance may be, for example, at least one selected from the group consisting of metal salts, cationic polymers, cationic surfactants, and cationic peptides. The cationic substance preferably is a metal salt, a cationic polymer, or a cationic surfactant, more preferably a cationic polymer or a cationic surfactant, and even more preferably includes both a cationic polymer and a metal salt, most preferably both a cationic polymer and sodium chloride.

### (Metal salt)

As the metal salt, any metal salt that generates metal ions when ionized in an aqueous solution can be used without any limitation. Even without a cationic functional group as described above, a metal salt that ionizes when it comes into contact with water becomes a cationic metal ion. Examples of metal ions include alkali metal ions, alkaline earth metal ions, and other metal ions, with alkali metal ions and alkaline earth metal ions being preferred. Examples of alkali metal ions include lithium ions, sodium ions, potassium ions, and the like. Examples of alkaline earth metal ions include magnesium ions, calcium ions, and the like. Examples of other metal ions include copper ions, and the like. Sodium ions are preferred as alkali metal ions, and magnesium ions are preferred as alkaline earth metal ions. The alkali metal salt is preferably sodium chloride or sodium citrate, and the alkaline earth metal salt is most preferably magnesium sulfate.

### (Cationic polymer)

In the present specification, the term "cationic polymer" means a polymer having a cationic functional group as a side chain. The cationic functional group as the side chain is preferably a quaternary ammonium group. The cationic polymer is preferably a cationic polymer having hexadimethrine bromide (CAS RN28728-55-4) or 2-methacryloyloxyethyl phosphorylcholine as constituent units, most preferably a cationic polymer having 2-methacryloyloxyethyl phosphorylcholine as constituent units, which exhibits a kinematic viscosity of 20 to 60 m²/s at 25 °C in the form of an aqueous solution with a concentration of 0.5 % by mass, exhibits a surface tension of 70 × 10 ⁻³ to 80 × 10⁻³ N/m at 25 °C in the form of an aqueous solution with a concentration of 0.1 % by mass, while having a mass average molecular weight (Mw) of 50 to 600 kDa. In this context, the cationic polymer having 2-methacryloyloxyethyl phosphorylcholine as constituent units is commercially available as Lipidure-BL502.

The kinematic viscosity η [m²/s] of the aqueous solution of the cationic polymer at a concentration of 0.5 % by mass at 25°C can be obtained by dividing the viscosity µ [Pa·s] by the density ρ [kg/m³], wherein the viscosity µ is determined by measuring an aqueous solution in which the cationic polymer is dissolved in water at a concentration of 0.5 % by mass with an Ubbelohde viscometer at 25 °C, and the density ρ is a density of the same aqueous solution at 25 °C.

The surface tension γ [mN/m] of a 0.1 % by mass aqueous solution of the cationic polymer at 25 °C can be determined by measurement at 25 °C by du Noüy ring method, Wilhelmy plate method, or drop weight method with respect to an aqueous solution in which the cationic polymer is dissolved in water at a concentration of 0.1 % by mass.

The mass average molecular weight (Mw) of the cationic polymer can be determined by gel permeation chromatography (GPC). Polyethylene glycol is used as a standard substance.

The lower limit of the mass average molecular weight may be, for example, 1,000, 2,000, 5,000, 10,000, 50,000, or 100,000. The upper limit of the mass average molecular weight may be, for example, 2,000,000, 1,000,000, 700,000, 100,000, or 70,000. The specific range of the mass average molecular weight may be, for example, 1,000 to 2,000,000, 2,000 to 1,000,000, 5,000 to 700,000, or 10,000 to 100,000. The mass average molecular weight can be measured by a method such as gel permeation chromatography.

### (Cationic surfactant)

In the present specification, the term "cationic surfactant" means a surfactant whose portion to which a hydrophobic group is bonded is positively charged when dissolved in an aqueous solution. Examples of cationic surfactants include alkylamine salt type surfactants and quaternary ammonium salt type surfactants.

Examples of alkylamine salt type surfactants include salts of amines having 1 to 3 alkyl groups each having 8 to 22 carbon atoms, such as monododecylamine, monooctadecylamine, dioctadecylamine, and triotadecylamine, with inorganic acids such as hydrochloric acid and sulfuric acid, or lower carboxylic acids such as acetic acid, lactic acid, and citric acid. Specific examples thereof include stearylamine acetate [CAS number: 2190-04-7, product name: Acetamin 86, manufactured by Kao Corporation].

Examples of quaternary ammonium salt type surfactants include salts of ammonium having 1 to 3 alkyl groups each having 8 to 22 carbon atoms, such as dodecyltrimethylammonium, octadecyltrimethylammonium, hexadecyltrimethylammonium, didecyldimethylammonium, and benzylmethyltetradecylammonium, with chlorine, bromine, etc. Specific examples thereof include lauryltrimethylammonium chloride [CAS number: 112-00-5, product name: Quartamin 24P, manufactured by Kao Corporation], dodecyltrimethylammonium bromide [CAS number: 1119-94-4, manufactured by Tokyo Chemical Industry Co., Ltd.], hexadecyltrimethylammonium bromide (also known as cetyltrimethylammonium bromide, abbreviation: CTAB) [CAS number: 57-09-0, manufactured by Tokyo Chemical Industry Co., Ltd.].

### (Cationic peptide)

The term "cationic peptide" means a peptide that exhibits cationicity when dissolved or dispersed in water. The cationic peptide may contain many basic amino acid residues (arginine (Arg), lysine (Lys), or histidine (His)) among the amino acid residues constituting the peptide. The cationic peptide is not particularly limited as long as it exhibits cationicity when dissolved or dispersed in water, and examples thereof include polyarginine, polylysine, and polyhistidine, which are polymers of the above-mentioned basic amino acid residues. As the cationic peptide, it is preferable to use cocodimonium hydroxypropyl hydrolyzed keratin (CAS RN68915-25-3). Cocodimonium hydroxypropyl hydrolyzed keratin is commercially available from Seiwa Kasei Co., Ltd. under the trade name Promois WK-HCAQ.

### (Step of bringing a sample into contact with a glass material)

The immunoassay method of the present invention includes a step of bringing a sample containing a detection target substance into contact with a glass material in the presence of a cationic substance. The order of contact when contacting the sample containing the cationic substance and the detection target substance with the glass material is not particularly limited. That is, the cationic substance can be brought into contact with the glass material before, simultaneously with, or after contacting with the sample containing the detection target substance. It is preferable that the sample containing the detection target substance is brought into contact with the glass material after or simultaneously with contacting the sample containing the detection target substance with the cationic substance, and it is more preferable that the sample containing the detection target substance is brought into contact with the glass material after contacting the sample containing the detection target substance with the cationic substance. An immunoassay method in which the sample containing the detection target substance is brought into contact with the glass material after contacting the sample containing the detection target substance with the cationic substance preferably includes, for example, the following steps (1) to (4).
(1) Step of bringing a sample containing a detection target substance into contact with a specimen diluent containing a cationic substance.
(2) Step of bringing the sample containing the detection target substance into contact with a glass material in the presence of the cationic substance.
(3) Step of bringing the detection target substance into contact with a first binding partner to which a labeling substance is directly or indirectly bound, thereby forming a first complex.
(4) Step of bringing the first complex into contact with a second binding partner to form a second complex.
(5) Step of detecting a signal derived from the labeling substance.

The step (2) may be a step (2-1) of filtering the sample diluted with the specimen diluent using a specimen filtration filter containing glass fibers, or a step (2-2) of bringing the sample diluted with the specimen diluent into contact with at least one selected from the group consisting of a sample pad, a third pad, and a conjugate pad of the immunochromatography test strip, each containing glass fibers. Further, the immunoassay method of the present invention can also include both step (2-1) and step (2-2) as the step (2). When the step (2-1) is included, the immunochromatography test strip in the step (2-2) does not need to contain a glass material.

The concentration of the cationic substance can be adjusted as appropriate depending on the type of target substance to be detected, the type of sample, and the type of immunoassay method. Examples of the concentration are given below. The following concentrations are applicable to both of the case where the cationic substance is included in the specimen diluent and the case where the cationic substance is disposed not in the specimen diluent but in the solid phase.

In the case of a metal salt, the concentration of the cationic substance is, for example, 0.1 to 1,000 mM, 1 to 750 mM, 2 to 750 mM, or 50 to 750 mM, based on the specimen diluent or the solution added to the solid phase. When the cationic substance is an alkali metal salt, the concentration of the cationic substance is 0.1 to 1,000 mM, 1 to 750 mM, 2 to 750 mM, 50 to 750 mM, or 200 to 750 mM. When the cationic substance is an alkaline earth metal salt, the concentration of the cationic substance is 0.1 to 1,000 mM, 1 to 500 mM, 1 to 250 mM, or 1 to 100 mM. Further, in the case of a cationic polymer, a cationic surfactant, or a cationic peptide, the concentration is, for example, 0.0001 to 20 % by mass, 0.0005 to 10 % by mass, 0.001 to 5 % by mass, 0.01 to 1 % by mass, or 0.01 to 0.5 % by mass, based on the specimen diluent or the solution added to the solid phase.

Even when the specimen diluent or the solution added to the solid phase contains a metal salt together with a cationic polymer, a cationic surfactant, or a cationic peptide, the concentration of each component may be within the above range.

### (Binding partner)

In the present specification, the term "binding partner" means a substance that binds to a detection target substance through an antigen-antibody reaction.

In the present invention, when the protein having an isoelectric point of 9.5 or higher as a detection target is an antibody, the binding partner may be an antibody or a protein other than antibodies. Further, in the present invention, when the protein having an isoelectric point of 9.5 or higher as a detection target is a protein other than antibodies, the binding partner is an antibody, namely a monoclonal antibody or a polyclonal antibody.

### (Antibody)

The antibody used in the immunoassay method of the present invention may be either a monoclonal antibody or a polyclonal antibody and can be produced according to known methods, but a monoclonal antibody is preferable. Monoclonal antibodies can be produced by, for example, isolating spleen cells or lymph node cells, which are antibody-producing cells, from a non-human mammal immunized with a detection target substance or a fragment thereof, fusing the isolated cells with a myeloma-derived cell line having high proliferative potential to prepare hybridomas, and purifying the antibodies produced by the hybridomas. Further, polyclonal antibodies can be obtained from the serum of animals immunized with a detection target substance or a fragment thereof. The fragment is a partial fragment of a detection target substance, and an antibody that binds to the fragment recognizes the detection target substance.

As the antibody, it is possible to use a fragment of an antibody having antigen-antibody reactivity as well as the whole antibody molecule. In the present specification, references to "antibody" also indicate fragments thereof. In addition to those obtained through the process of immunizing animals as described above, it is also possible to use those obtained using genetic recombination technology or chimeric antibodies. Fragments of antibodies are preferably functional fragments, such as F(ab') ₂, Fab', scFv and the like. These fragments can be prepared by treating the antibody obtained as described above with a protease (e.g., pepsin or papain), or cloning the DNA of the antibody and expressing it in a culture system using E. coli or yeast.

The immunoassay can be performed with only a single type of the antibody by, for example, adopting the competitive method described below. However, it is preferable to construct a sandwich system using two types of antibodies. In this instance, the two antibodies preferably recognize different epitopes. To recognize different epitopes in this context means that the amino acid sequences recognized as epitopes by the two monoclonal antibodies do not overlap. Using "two types" of the antibodies requires no more than using "at least two types" of the antibodies, and does not exclude embodiments using more than two types of the antibodies from the scope of the present invention.

When constructing a sandwich system, it is preferable that at least one of the two types of antibodies is a solid-phase antibody, and at least one is preferably a labeled antibody. In the present specification, the "solid-phase antibody" means an antibody directly or indirectly immobilized on a solid phase. In the present specification, the "labeled antibody" means an antibody that is directly or indirectly labeled with a conventional labeling substance well known in the art described below when measuring a signal attributable to the labeling substance.

The present specification includes descriptions indicating that an antibody or an antibody fragment thereof "reacts with", "recognizes" or "binds to" a specific substance or amino acid sequence, which however are used interchangeably. Whether or not an antibody "reacts with" an antigen (compound) can be determined through antigen-immobilized ELISA, competitive ELISA, sandwich ELISA, or the like. Alternatively, a method using the principle of surface plasmon resonance (SPR method) can also be used. The SPR method can be performed using equipment, sensors and reagents commercially available under the name Biacore.

The use of an antibody capable of directly binding to the detection target substance in the immunoassay assay method of the present invention also enables the measurement of amount of the antibody bound to detection target substance. In the present specification, an antibody which is bound to the detection target substance and has a labeling substance bound thereto is referred to as a "secondary antibody". This secondary antibody may be used to indirectly bind the labeling substance to an antibody to which the detection target substance is bound.

### (Solid phase)

The shape of the solid phase is not particularly limited, and any appropriate shape may be chosen, for example, from a plate shape (e.g., microplate or membrane), a bead or particulate shape (e.g., latex particles, magnetic particles), or a cylindrical shape (e.g., test tube). For example, a binding partner having a solid-phase bound thereto can be produced by allowing a binding partner to physically adsorb or chemically bind to a solid phase (optionally via an appropriate spacer). The solid phase to be used may be a solid phase composed of a polymer substrate such as a polystyrene resin, an inorganic substrate such as glass, a polysaccharide substrate such as cellulose or agarose, or the like, but it is preferable to use a glass material. In the present specification, the process of physically or chemically supporting a binding partner on a solid phase or the state of a binding partner being supported on a solid phase is sometimes referred to as "immobilization" or " solid-phase immobilization".

### (Labeling substance)

In the present invention, it is preferable that a labeling substance is bound indirectly or directly to a binding partner, preferably an antibody. By measuring the intensity of the signal generated by the labeling substance, the amount of a detection target substance in the sample can be measured.

Examples of labeling substances for preparing a binding partner having a labeling substance bound thereto, which is preferably a labeled antibody, include metal complexes, enzymes, insoluble particles, fluorescent substances, chemiluminescent substances, biotin, avidin, radioactive isotopes, colloidal gold particles, and colored latex. The method used for binding the labeling substance to the binding partner may be physical adsorption, glutaraldehyde method, maleimide method, pyridyl disulfide method, or periodic acid method, which are available to those skilled in the art. When an enzyme such as horseradish peroxidase (HRP) or alkaline phosphatase (ALP) is used as a labeling substance, enzymatic activity can be measured using a specific substrate for the enzyme. For example, as the substrate for measuring the enzymatic activity, o-phenylenediamine (OPD) or 3,3',5,5'-tetramethylbenzidine (TMB) can be used when the enzyme is HRP, and p-nitrophenyl phosphate can be used when the enzyme is ALP. When biotin is used as a labeling substance, the binding partner may be labeled with biotin and reacted with avidin or streptavidin labeled with an enzyme, dye, or fluorescent label (preferably HRP).

The term "assay", "detection" or "measurement" also encompasses proving the presence of the detection target substance and quantification of the detection target substance.

### (Immunoassay method)

The "immunoassay method" is a method of measuring the level of a substance contained in a sample using the reaction between an antigen and an antibody. The term "level" encompasses the amount, concentration, or determination of presence or absence of a substance.

Hereinbelow, embodiments of the immunoassay method in which the binding partner is an antibody are described.

In the immunoassay method of the present invention, it is preferable to use two types of antibodies that recognize different epitopes. For convenience, one of the antibodies may be referred to as "first antibody", while the other one of the antibodies may be referred to as "second antibody".

Representative examples of the immunoassay method of the present invention using an antibody as the binding partner include the following first embodiment and second embodiment. The first embodiment is preferable because the sensitivity is higher.

The first embodiment of the immunoassay method of the present invention includes the following steps (1) to (5):
(1) a step of bringing a sample containing a detection target substance into contact with a specimen diluent containing a cationic substance,
(2) a step of bringing the sample diluted with the specimen diluent into contact with a glass material in the presence of the cationic substance,
(3) a step of contacting the detection target substance with a first antibody to form a first complex,
(4) a step of contacting the first complex with a second antibody to form a second complex (containing the detection target substance, the labeling substance bound to the first antibody or the second antibody, the first antibody, and the second antibody), and
(5) a step of measuring a signal derived from the labeling substance.

The characteristic feature of the first aspect is that it includes a step of bringing a sample containing a detection target substance into contact with a specimen diluent containing a cationic substance. In the first aspect, after the sample containing the detection target substance and the cationic substance come into contact, a glass material (e.g., a specimen filtration filter containing glass fibers, a glass fiber pad, a porous membrane, an immunoplate, etc.) and the sample diluted with the specimen diluent come into contact.

The step (2) is preferably:
(2-1) a step of filtering the sample diluted with the specimen diluent in the presence of the cationic substance, using a specimen filtration filter containing glass fibers.

Alternatively, the step (2) may be:
(2-2) a step of bringing the sample diluted with the specimen diluent into contact with at least one selected from the group consisting of a sample pad, a third pad, and a conjugate pad of an immunochromatography test strip, each containing glass fibers.

Further, the immunoassay method of the present invention can also include both step (2-1) and step (2-2) as the step (2). When the step (2-1) is included, the immunochromatography test strip in the step (2-2) does not need to contain a glass material, but preferably contains a glass material.

Further, one of the first antibody and the second antibody is a labeled antibody, while the other is a solid-phase antibody. It is preferable that the first antibody is a labeled antibody and the second antibody is a solid-phase antibody.

For signal measurement, a measurement method well known in the art can be employed depending on the labeling substance. The signal measurement may be performed using a measuring instrument, or may be performed visually.

The second embodiment of the immunoassay method of the present invention may include the following steps (1) to (4).
(1) Step of bringing the sample containing the detection target substance into contact with a glass material in the presence of the cationic substance.
(2) Step of contacting the detection target substance with a first antibody to form a first complex.
(3) Step of contacting the first complex with a second antibody to form a second complex (containing the detection target substance, the labeling substance, the first antibody, and the second antibody).
(4) Step of measuring a signal derived from the labeling substance.

The characteristic feature of the second aspect is that it does not include the step (1) of the first aspect, i.e., a step of bringing a sample containing a detection target substance into contact with a specimen diluent containing a cationic substance. In the second aspect, the glass material (e.g., a porous membrane formed of glass fibers, an immunoplate, etc.) and the cationic substance are in contact with each other before the sample containing the detection target substance contacts the glass material. It is preferable that the step (1) is (1-1) a step of bringing the sample containing the detection target substance into contact with a sample pad containing glass fibers of an immunochromatography test strip in the presence of the cationic substance.

Further, one of the first antibody and the second antibody is a labeled antibody, while the other is a solid-phase antibody. It is preferable that the first antibody is a labeled antibody and the second antibody is a solid-phase antibody.

The immunoassay method of the present invention may include, if necessary, a step of comparing the intensity of the obtained signal with a first threshold. The first threshold may be appropriately set in consideration of sensitivity and type of the sample, etc.

The immunoassay method of the present invention may include a step of determining that the subject is infected with a respiratory infection virus when the signal intensity is lower (higher) than the first threshold, or a step of determining that the subject is not infected with a respiratory infection virus when the signal intensity is higher (lower) than the first threshold.

Examples of the immunoassay method of the present invention include, but not limited to, immunochromatography, electrochemiluminescence immunoassay (ECL method), enzyme-linked immunosorbent assay (ELISA), latex immunoturbidimetric assay (LTIA method), chemiluminescence immunoassay, and immunofluorescence. The immunoassay method of the present invention is preferably immunochromatography.

The immunoassay method of the present invention can be an in vivo or in vitro immunoassay method. Further, a sensitizer may also be used to enhance sensitivity.

The procedure and principle of measurement are explained below taking immunochromatography as an example. The following descriptions are presented for illustrative purpose only with respect to the measurement procedure and principle for one embodiment of the present invention, and by no means limit the scope of the present invention. Further, although the embodiments below are described assuming that the binding partner is an antibody, the binding partner may be a protein other than an antibody.

### (Immunochromatography)

Immunochromatography is an immunoassay method that utilizes the behavior that a labeled antibody bound to a detection target substance and a labeled antibody flow on a membrane. A general principle in measuring a target substance to be detected by the immunochromatography is as follows.

An antibody against a detection target substance is immobilized on an insoluble membrane carrier, which is a chromatographic medium, to prepare a detector section, which is a stationary phase. Then, a conjugate (labeling substance sensitized by an antibody capable of binding to the target substance to be detected) is used as a mobile phase. The target substance to be detected and the conjugate as a mobile phase are allowed to react specifically, and the resulting the target substance bound to the conjugate is allowed to react, in the detector unit as a stationary phase, specifically with the antibody immobilized on the detector unit.

The measurement procedure and principle when the immunochromatography is employed as the immunoassay method of the present invention are as follows.
(1) A sample supply section on a sample pad is brought into contact with a sample containing a detection target substance.
(2) The detection target substance in the sample and a conjugate come into contact to form a first complex (including the detection target substance, the labeling substance, and the first antibody). The conjugate is a labeling substance bound to the first antibody.
(3) The first complex and a second antibody immobilized on the detector section come into contact to form a second complex (including the detection target substance, the labeling substance, the first antibody, and the second antibody).
(4) The intensity of the signal derived from the labeling substance contained in the conjugate is measured to confirm the formation of the second complex.

In this instance, the sample pad is preferably formed of glass fibers.

Further, the immunoassay method of the present invention preferably adopts the following procedures before the measurement procedure (1).
- A sample containing the detection target substance is brought into contact with a specimen diluent containing a cationic substance.
- The sample diluted with the specimen diluent is filtered with a specimen filtration filter in the presence of the cationic substance.

Examples of labeling substances include colloidal gold particles, colloidal platinum particles, color latex particles, magnetic particles, and the like. Among these, colloidal gold particles are preferable.

### 2. Immunochromatography kit

### (Immunochromatography test strip)

The immunochromatography kit of the present invention includes an immunochromatography test strip. Hereinbelow, the immunochromatography test strip may be simply referred to as a test strip.

The test strip of the present invention includes a glass material to be contacted with a sample containing a protein with an isoelectric point of 9.5 or higher. The glass material is preferably at least one selected from the group consisting of a specimen filtration filter, a sample pad, a third pad, and a conjugate pad.

The test strip of the present invention includes a cationic substance to be contacted with a sample containing a protein with an isoelectric point of 9.5 or higher. The cationic substance is contained in the specimen diluent and/or the test strip, preferably contained in at least one selected from the group consisting of the specimen diluent, the sample pad, the third pad, and the conjugate pad, and most preferably contained in the specimen diluent.

FIG. 4 shows the configuration of an embodiment of an immunochromatography test strip that can be used in the present invention. The test strip 200 shown in FIG. 4 has a backing sheet 201, a conjugate pad 202, an absorbent pad 203, an insoluble membrane carrier 204, a sample pad 208, and a third pad 209. In the test strip 200, the sample pad 208, the third pad 209, the conjugate pad 202, the insoluble membrane carrier 204, and the absorbent pad 203 are arranged in this order from upstream to downstream in the flow direction of the sample. Each pad is arranged so as to at least partially overlap the pads positioned above and below. A test line (detector section) 206 and a control line 207 are disposed on the insoluble membrane carrier 204. Preferably, a cationic substance is added to at least one of the sample pad 208, the third pad 209, and the conjugate pad 202.

### (Test strip)

Preferably, the test strip is placed on a solid support (backing sheet) such as a plastic adhesive sheet. The solid support is composed of a material that does not impede capillary flow of the sample and the conjugate. Alternatively, the test strip may be immobilized on the solid support using an adhesive or the like.

The test strip includes a sample pad and an insoluble membrane carrier, and may further have other configurations depending on the measurement conditions and the sample. Examples of other configurations include a conjugate pad, a third pad, and an absorbent pad. Preferably, at least one of the sample pad, conjugate pad, and third pad of the test strip includes glass fibers. However, when the sample is filtered with a specimen filtration filter containing glass fibers before contacting the sample with the sample pad, none of the sample pad, conjugate pad, and third pad of the test strip may contain glass fibers.

### (Sample pad)

In the test strip of the present invention, a pad-shaped porous material can be used as the sample pad. The sample pad has a sample supply section in a part thereof. Further, a porous material section is present downstream of the sample supply section.

The sample supply section is a part that supplies a sample that may contain a detection target substance. This sample supply section is formed in a part of the porous material, and the sample supply section is on the upstream side of the sample pad. The sample pad is deployed so as to establish fluid communication with the insoluble membrane carrier.

### (Conjugate pad)

In the present specification, the term "conjugate pad" means a pad-shaped porous material impregnated with a conjugate and dried. The conjugate pad has the function of forming a complex between the conjugate and the detection target substance when the sample passes through the conjugate pad. The above-mentioned sample supply section and conjugate pad may also be placed on the same porous material.

### (Insoluble membrane carrier)

The insoluble membrane carrier that can be used in the present invention has at least one detector section for capturing and detecting a detection target substance. It is preferable that the detector section has a linear shape. It is preferable that the detector section has immobilized thereon a binding partner, preferably an antibody, which immunologically reacts with a detection target substance. Immobilization of an antibody that immunologically reacts with a detection target substance to an insoluble membrane carrier can be carried out by a conventionally known method. A third pad can also be placed between the sample supply section and the insoluble membrane carrier.

Examples of porous materials forming the sample pad, third pad, conjugate pad, and insoluble membrane carrier include pads composed of paper, cellulose mixture, nitrocellulose, polyester, acrylonitrile copolymer, glass fibers, or nonwoven fibers such as rayon, etc. Particularly, the sample pad and the conjugate pad are preferably pads formed of glass fibers or polyester, and the insoluble membrane carrier is preferably a membrane formed of nitrocellulose.

### (Absorbent pad)

In the test strip of the present invention, an absorbent pad is preferably provided at the downstream end of the insoluble membrane carrier. The absorbent pad is a part having liquid absorbency that controls the development of the sample by absorbing the sample that has been transferred and passed through the insoluble membrane carrier. As the absorbent pad, a known absorbent pad conventionally used in test strips can be used, and for example, filter paper can be used.

### (Specimen diluent)

The immunochromatography kit of the present invention includes a specimen diluent. Although it is preferable that the specimen diluent contains a cationic substance at the time of sale, the immunochromatography kit may contain a cationic substance separately from the specimen diluent. In such case, a person performing immunochromatography can mix the cationic substance and the specimen diluent. In this context, the cationic substance being "contained in the specimen diluent" should be regarded as encompassing an embodiment in which the cationic substance is dissolved in the specimen diluent at the time of sale, and an embodiment in which the cationic substance is not dissolved in the sample dilution liquid at the time of sale, but is dissolved in the specimen diluent by a person performing immunochromatography at the time of measurement.

When the cationic substance is a metal salt, the concentration of the cationic substance is, for example, 0.1 to 1,000 mM, 1 to 750 mM, 2 to 750 mM, or 50 to 750 mM, based on the specimen diluent.

When the cationic substance is a cationic polymer, a cationic surfactant, or a cationic peptide, the concentration of the cationic substance is, for example, 0.0001 to 20 % by mass, 0.0005 to 10 % by mass, 0.001 to 5 % by mass, 0.01 to 1 % by mass, or 0.01 to 0.5 % by mass, based on the specimen diluent.

Even when the specimen diluent contains a metal salt together with a cationic polymer, a cationic surfactant, or a cationic peptide, the concentration of each component may be within the above range.

Preferably, the specimen diluent contains a buffer. The term "buffer" means a solution having a pH-buffering effect.

Non-limiting examples of buffers that can be used as appropriate in the present invention include known buffers such as PBS (phosphate buffered saline), MES (2-(N-morpholino)ethanesulfonic acid), PIPES (piperazine-N,N'- bis(2-ethanesulfonic acid), ACES (N-(2-acetamido)-2-aminoethanesulfonic acid), ADA (N-(2-acetamido)iminodiacetic acid), Bis-Tris(2,2-bis(hydroxyethyl)-(iminotris-(hydroxymethyl)-methane), Tris (tris(hydroxymethyl)aminomethane), MOPS (3-morpholinopropanesulfonic acid), HEPES (4-(2-hydroxyethyl)-1-piperazineethanesulfonic acid), a citric acid buffer, a glycine buffers, a borate buffer, and a phosphate buffer. The buffer used in the present invention is preferably a phosphate buffer.

The immunochromatography kit of the present invention may also include other test reagents and/or an instruction manual, etc., such as a specimen filtration filter containing glass fibers, a cotton swab for sample collection, a standard antigen substance, and a quality control antigen sample. The kit of the present invention preferably includes a specimen filtration filter containing glass fibers.

### 3. Filtration method for a sample containing a protein with an isoelectric point of 9.5 or higher

The filtration method of the present invention can prevent a detection target substance from being adsorbed to a specimen filtration filter. Therefore, the filtration method of the present invention can prevent a detection target substance from being adsorbed to a specimen filtration filter before the measurement or detection in a wide range of applications such as immunoassays and nucleic acid detection.

### 4. Use for suppressing adsorption of a protein with an isoelectric point of 9.5 or higher to glass materials

In the immunoassay method of the present invention, the above-mentioned cationic substance is suitable for use in suppressing adsorption of a protein with an isoelectric point of 9.5 or higher to a glass member.

### [Examples]

Hereinbelow, the present invention is described in detail with reference to examples. However, the examples described below should not be construed as limiting the present invention. In the following description, the unit "%" refers to "% by mass", unless otherwise specified.

### [Preparation of anti-SARS-CoV-2 monoclonal antibody]

Anti-SARS-CoV-2 monoclonal antibodies used in the following tests were obtained by immunizing mice with recombinant SARS-CoV-2 nuclear proteins as an antigen and using a method commonly used by those skilled in the art to produce monoclonal antibodies.

### [Preparation of immunochromatography test strip for SARS-CoV-2 measurement]

### Preparation of colloidal gold particle-labeled anti-SARS-CoV-2 monoclonal antibody (conjugate)

1) Preparation of colloidal gold-labeled antibody solution
   1 mL of phosphate buffer containing 25 µg/mL of anti-SARS-CoV-2 antibody was added to 20 mL of 1 OD/mL-colloidal gold solution, and the resulting mixture was stirred at room temperature for 10 minutes. Subsequently, 2 mL of 10 % BSA solution was added to the colloidal gold solution and the resulting was stirred at room temperature for 5 minutes. The obtained solution was centrifuged at 10,000 rpm for 45 minutes at 10 °C, and the supernatant was removed. The residue was suspended in Conjugate Dilution Buffer (Scripps) to obtain a gold colloid-labeled antibody solution.
2) Preparation of conjugate pad

The colloidal gold-labeled antibody solution prepared in 1) was diluted to 4 OD/mL with a 1.33 % casein, 4 % sucrose solution (pH 7.5) to prepare a conjugate solution. The conjugate solution was applied in lines onto a glass fiber pad and dried to obtain a conjugate pad.

### 3) Preparation of antibody-immobilized membrane

PBS containing 0.75 mg/mL anti-SARS-CoV-2 antibody and 2.5 % sucrose was prepared and used as a test line coating solution. PBS containing 1.0 mg/mL goat antimouse IgG monoclonal antibody and 2.5 % sucrose was prepared and used as a control line coating solution. Using a dispenser for immunochromatography "XYZ3050" (manufactured by BioDot), the test line coating solution and the control line coating solution were each applied on a nitrocellulose membrane at 1.0 µL/cm and dried to obtain an antibody-immobilized membrane.

### 4) Fabrication of test device

The antibody-immobilized membrane, the conjugate pad, and an absorbent pad were attached to a plastic adhesive sheet, and the resultant was cut into a 5 mm-width strip to obtain an immunochromatography test strip for measuring SARS-CoV-2.

The configuration of this immunochromatography test strip is shown in FIG. 3. The immunochromatography test strip 100 shown in FIG. 3 includes a backing sheet 101 formed of a plastic adhesive sheet, a conjugate pad 102, an absorbent pad 103, and an insoluble membrane carrier 104. In the immunochromatography test strip 100, the conjugate pad 105, the insoluble membrane carrier 104, and the absorbent pad 103 are arranged in this order from upstream to downstream in the flow direction of the sample. A test line (detector section) 106 and a control line 107 are provided on the insoluble membrane carrier 104.

### [Analysis Example 1]

### Evaluation of the effect of antigen solution filtration on sensitivity

Samples were prepared by adding the following antigens to the specimen diluent attached to RapidTesta FLU/NEXT (Sekisui Medical Co., Ltd.).
Influenza A virus antigen: derived from Kitakyusyu 159/93 strain
Influenza B virus antigen: derived from Lee 40 strain
RS virus antigen: RSV Antigen, Long strain inactivated antigen (Meridian)
Adenovirus antigen: Adenovirus Antigen (Fitzgerald)
SARS-CoV-2 antigen: SARS-CoV-2 Nucleocapsid Recombinant Protein (Icosagen)

A portion of the sample was filtered using a specimen filtration filter attached to RapidTesta FLU/NEXT to obtain a filtered sample. The sample and filtered sample were measured under the conditions shown in Table 1, and the absorbance of the test line was calculated using a RapidTesta Reader (Sekisui Medical Co., Ltd.).

**[Table 1]**

| Antigen | Measurement reagent | Antigen liquid amount [µL] | Measurem ent time [min.] |
|---|---|---|---|
| Influenza A virus | RapidTesta FLU/NEXT test device | 120 | 15 |
| Influenza B virus | RapidTesta FLU/NEXT test device | 120 | 15 |
| RS virus | RapidTesta RSV-Adeno/NEXT test device | 120 | 10 |
| Adenovirus | RapidTesta RSV-Adeno/NEXT test device | 120 | 10 |
| SARS-CoV-2 | Immunochromatography test strip for SARS-CoV-2 measurement | 120 | 15 |

The filtration recovery rate when the sample was filtered through the specimen filtration filter was calculated by the following formula. Recovery rate (%) = absorbance in the measurement of filtered sample/absorbance in the measurement of sample (without filtration) x 100

Table 2 shows the filtration recovery rate for each antigen.

For the influenza A virus antigen, influenza B virus antigen, RS virus antigen, and adenovirus antigen, the filtration recovery rates were all within 100 ± 15 %, suggesting no influence of specimen filtration using the specimen filtration filter on the measurements. On the other hand, the filtration recovery rate for the SARS-CoV-2 antigen was 47 %, which is significantly lower than the values for other antigens, thus suggesting that the measurement sensitivity decreases as a result of specimen filtration using the specimen filtration filter.

**[Table 2]**

| Antigen | Filtration recovery rate [%] |
|---|---|
| Influenza A virus | 88 |
| Influenza B virus | 99 |
| RS virus | 91 |
| Adenovirus | 101 |
| SARS-CoV-2 | 47 |

### [Analysis Example 2]

### Evaluation of antigen adsorption to filter member

A sample was prepared by adding SARS-CoV-2 antigen to the specimen diluent provided with RapidTesta FLU/NEXT. An opening-side filter holder (formed of polyvinyl alcohol (PVA)), a filter member (formed of glass fibers), and a tip-side filter holder (formed of PVA) were removed from the specimen filtration filter attached to the RapidTesta FLU/NEXT, and these were immersed and held in 500 µL of sample for 10 minutes to obtain a filter member immersion sample. 120 µL each of the sample and the filter member immersion sample were dropped onto an immunochromatography test strip for SARS-CoV-2 measurement. Fifteen minutes after dropping the antigen solution, the absorbance of the test line was measured using a RapidTesta Reader (Sekisui Medical Co., Ltd.).

The antigen recovery rate when the specimen filtration filter member was immersed in the sample was calculated by the following formula. Recovery rate (%) = absorbance in the measurement of filter member immersion sample/absorbance in the measurement of sample (without immersion of filter member) x 100

Table 3 shows the antigen recovery rate when each member was immersed in the sample. The antigen recovery rate when the PVA filter holder was immersed in the sample was about 70 %, whereas the recovery rate when the glass fiber filter was immersed in the sample was about 10 %.

**[Table 3]**

| Specimen filtration filter member | Material of filter member | Recovery rate [%] |
|---|---|---|
| Opening-side filter holder | PVA | 74 |
| Filter member | Glass fiber | 10 |
| Tip-side filter holder | PVA | 69 |
| Filter member as a whole | PVA, glass fiber | 9 |

### [Analysis Example 3]

### Evaluation of antigen adsorption to conjugate pad material

The same procedure as in Analysis Example 2 was followed, except that the filter member in Analysis Example 2 was changed to the conjugate pad material shown in Table 4, and 140 mm² of the conjugate pad material was immersed per 120 µL of the sample.

The results are shown in Table 4.

With the polyester fiber pad, there was no decrease in the recovery rate due to immersion of the pad material in the sample, whereas with the glass fiber pad, the recovery rate decreased.

**[Table 4]**

| Material of conjugate pad | Recovery rate [%] |
|---|---|
| Glass fiber pad 1 | 51 |
| Glass fiber pad 2 | 81 |
| Polyester fiber pad 1 | 102 |
| Polyester fiber pad 2 | 104 |

From the results of Analysis Examples 2 and 3, it was presumed that contact between the antigen and the glass fibers decreased the amount of antigen subjected to subsequent immunochromatography, resulting in a decrease in the recovery rate.

In this context, Table 5 shows the isoelectric points of proteins contained in the respective antigens used in Analysis Example 1 and antigens similar thereto and detected by the respective measurement reagents. The isoelectric points of the proteins were calculated by inputting UniProtKB into Expaxy Compute pI/Mw (https://www.expasy.org/resources/compute-pi-mw). Table 5 shows that only the isoelectric point of the nucleocapsid protein of SARS-CoV-2 is as high as 9.5 or higher. Glass fibers have a negative charge when contacted with a neutral specimen diluent, whereas the nucleocapsid protein of SARS-CoV-2, which has an isoelectric point of 10.07, has a positive charge. Therefore, the nucleocapsid protein of SARS-CoV-2 is considered to be more easily adsorbed to glass fibers than other antigenic proteins with an isoelectric point of less than 9.5. This has led to an expectation that, in order to suppress the decrease in measurement sensitivity due to contact between the glass fibers and the antigen solution, it would be effective to allow a positively charged compound to coexist when the glass fibers and the antigen solution come into contact.

**[Table 5]**

| Antigen | Protein detected by reagent | UniProtKB | Isoelectric point |
|---|---|---|---|
| Influenza A virus | Nucleocapsid protein | 091743 (NCAP_193A0) | 9.35 |
| Influenza B virus | Nucleocapsid protein | P04665 (NCAP_INBLE) | 9.40 |
| RS virus | Fusion glycoprotein F0 | P03420 (FUS_HRSVA) | 9.10 |
| Adenovirus | Hexon protein | P03277 (CAPSH_ADE02) | 5.06 |
| SARS-CoV-2 | Nucleocapsid protein | P0DTC9 (NCAP_SARS2) | 10.07 |
| MERS-CoV | Nucleocapsid protein | K9N4V7 (NCAP_MERS1) | 10.05 |
| SARS-CoV | Nucleocapsid protein | P59595 (NCAP_SARS) | 10.11 |

### [Experimental Example 1]

### Effect of cationic substances on SARS-CoV-2 antigen measurement sensitivity

The cationic substances listed in Table 6 and SARS-CoV-2 nucleocapsid recombinant protein (Icosagen) were added to the specimen diluent attached to the RapidTesta FLU/NEXT at a concentration of 450 TCID ₅₀/mL, thereby preparing samples. 120 µL each of the samples were dropped onto an immunochromatography test strip for SARS-CoV-2 measurement. Ten minutes after dropping the antigen solution, the absorbance of the test line was measured using a RapidTesta Reader (Sekisui Medical Co., Ltd.). The configuration of the immunochromatography test strip used was the same as shown in FIG. 3.

The results are shown in Table 6.

Under all conditions in which the cationic substance was added, the absorbance during SARS-CoV-2 antigen measurement increased compared to the conditions in which the additive was not added. The reason for this is presumably as follows. Since the immunochromatography test strip for SARS-CoV-2 measurement contained glass fibers, the cationic substance served to suppress adsorption of the SARS-CoV-2 antigen to the glass fibers.

**[Table 6]**

| Cationic substance | | | | Absorbance [mAbs] |
|---|---|---|---|---|
| Type | | Final concentration | | |
| None | | - | | 49.8 |
| Metal salt | NaCl | 150 mM | | 71.9 |
| | NaCl | 250 mM | | 81.4 |
| | Trisodium citrate | 10 mM | | 72.2 |
| | Trisodium citrate | 100 mM | | 102.4 |
| | MgSO₄ | 5 mM | | 94.7 |
| | MgSO₄ | 10 mM | | 111.6 |
| Cationic polymer | hexadimethrine bromide | 0.0005 % | | 65.6 |
| | hexadimethrine bromide | 0.001 % | | 103.7 |
| | hexadimethrine bromide | 0.01 % | | 104.3 |
| | hexadimethrine bromide | 0.025 % | | 84.0 |
| | Lipidure-BL502 | 0.01 % | | 78.7 |
| | Lipidure-BL502 | 0.05 % | | 90.2 |
| | Lipidure-BL502 | 0.20 % | | 106.0 |
| Cationic surfactant | Quartamin 24P | 0.50 % | | 116.7 |
| | Quartamin 24P | 1.00 % | | 169.4 |
| | CTAB | 0.05 % | | 143.5 |
| | CTAB | 0.10 % | | 97.4 |
| Cationic peptide | Promois WK-HCAQ-NA | 0.50 % | | 72.6 |
| | Promois WK-HCAQ-NA | 1.00 % | | 69.5 |
| Cationic polymer + Metal salt | Lipidure-BL502 NaCl | 0.05 % | | 244.3 |
| | | 250 mM | | |

### Effect of cationic substances on SARS-CoV-2 antigen recovery rate

The cationic substances listed in Table 7 and SARS-CoV-2 nucleocapsid recombinant protein (Icosagen) were added to the specimen diluent attached to the RapidTesta FLU/NEXT at a concentration of 450 TCID ₅₀/mL, thereby preparing samples.

The sample was filtered using a specimen filtration filter attached to RapidTesta FLU/NEXT to obtain a filtered sample.

120 µL each of the sample and the filtered sample were dropped onto an immunochromatography test strip for SARS-CoV-2 measurement prepared by the method described in Preparation Example 1. Ten minutes after dropping the antigen solution, the absorbance of the test line was calculated using a RapidTesta Reader (Sekisui Medical Co., Ltd.). The measurement was performed three times for each sample. The configuration of the immunochromatography test strip used was the same as shown in FIG. 3.

The filtration recovery rate when the sample was filtered through the specimen filtration filter was calculated by the following formula. Recovery rate (%) = absorbance in the measurement of filtered sample/absorbance in the measurement of sample (without filtration) x 100

**[Table 7]**

| Cationic substance | | No additive | | 0.05% Lipidure-BL502 | | 250mM NaCl | | 0.05% Lipidure-BL502 + 250mM NaCl | |
|---|---|---|---|---|---|---|---|---|---|
| Filtration | | Not done | Done | Not done | Done | Not done | Done | Not done | Done |
| Absorbance [mAbs] | Measurement 1 | 38.9 | 9.8 | 85.0 | 43.8 | 85.8 | 75.9 | 116.5 | 120.0 |
| | Measurement 2 | 55.0 | 12.4 | 93.3 | 35.8 | 102.2 | 67.8 | 120.2 | 121.7 |
| | Measurement 3 | 29.7 | 15.3 | 103.0 | 47.7 | 93.4 | 80.9 | 128.7 | 121.8 |
| | Average value | 41.20 | 12.50 | 93.76 | 42.43 | 93.82 | 74.84 | 121.82 | 121.17 |
| Antigen recovery rate [%] | | - | 30.3 | - | 45.3 | - | 79.8 | - | 99.5 |

The antigen recovery rate was improved by performing filtration using the glass fiber filter in the copresence of the cationic substance.

### [Experimental Example 2]

### Effect of cationic substances on the sensitivity of antigen measurements

The same operation as in Experimental Example 1 was performed using the cationic substances listed in Table 8. In addition, as described below, the effect of the cationic substances on the measurements for influenza A virus antigen and influenza B virus antigen was evaluated. The cationic substances listed in Table 8 and an influenza A virus antigen (6.25 × 10³TCID₅₀/mL) or an influenza B virus antigen (1.0 × 10⁵TCID₅₀/mL) were added to the specimen diluent attached to the RapidTesta FLU/NEXT, thereby preparing samples. 120 µL of each of the samples were dropped onto a RapidTesta FLU/NEXT test device. Ten minutes after dropping the antigen solution, the absorbance of the test line was calculated using a RapidTesta Reader (Sekisui Medical Co., Ltd.). The results are shown in Table 8. In the measurement of SARS-CoV-2 antigen, the measurement sensitivity was improved by the cationic substance, whereas for the influenza antigen, almost no variation in measurement sensitivity was observed, or a decrease in measurement sensitivity was observed.

**[Table 8]**

| Cationic substance | | Absorbance in antigen measurement [mAbs] | | |
|---|---|---|---|---|
| Type | Final concent ration | SARS-CoV-2 | FLU A | FLU B |
| None | - | 18.9 | 25.8 | 32.3 |
| Hexadimethrine bromide | 0.01 % | 77.1 | 26.9 | 25.0 |
| Quartamin 24P | 0.50 % | 64.2 | 27.0 | 30.6 |
| Quartamin 86P | 0.25 % | 67.3 | 24.1 | 0.0 |
| CTAB | 0.25 % | 59.9 | 15.9 | 20.0 |
| Promois WK-HCAQ-NA | 5.00 % | 40.5 | 26.8 | 27.2 |

### [Experimental Example 3]

### Effect of cationic substances on clinical sample measurement sensitivity

The cationic substances listed in Table 9 were added to the specimen diluent attached to the RapidTesta FLU/NEXT to prepare cation-added specimen diluents. To 20 pieces of SARS-CoV-2 Validation Panel (Boca Biolistics), Universal Transport Medium (BD) was added to prepare samples. A specimen was collected with a sterile cotton swab attached to the RapidTesta FLU/NEXT, and suspended respectively in the specimen diluent attached to the RapidTesta FLU/NEXT and the cation-added specimen diluent. The specimen diluent attached to the RapidTesta FLU/NEXT contains a phosphate buffer. The diluent in which the specimen was suspended was filtered using a specimen filtration filter attached to the RapidTesta FLU NEXT, thereby obtaining a sample. 120 µL of the filtered sample was dropped onto an immunochromatography test strip for SARS-CoV-2 measurement, and ten minutes after dropping the sample, the absorbance of the test line was measured using a RapidTesta Reader (Sekisui Medical Co., Ltd.).

RNA was extracted from the same specimen using QIAmp (registered trademark) Viral RNA mini Kit (QIAGEN). RT-PCR was performed following "Pathogen Detection Manual 2019-nCoV Ver. 2.9.1", using N set No. 2 primers, and the copy number of SARS-CoV-2 per immunochromatography test was calculated.

The results are shown in Table 9.

Evaluation was made as follows, based on the absorbance of the test line.
-: less than 5 mAbs and negative
+: less than 20 mAbs and positive
++: Positive with 20 mAbs or more and less than 100 mAbs
+++: Positive with 100 mAbs or more

For all specimens that tested positive with the immunochromatography test strip for SARS-CoV-2 measurement, the absorbance of the test line was higher when using the cation-added specimen diluent than when using the specimen diluent without cations. Further, specimen 1, which was negative in the measurement using the specimen diluent, was determined to be positive when using the specimen diluent to which 0.05 % Lipidure-BL502 and 250mM NaCl had been added. Specimens 2 to 6, which were negative when using any of the diluents, were all low copy number specimens.

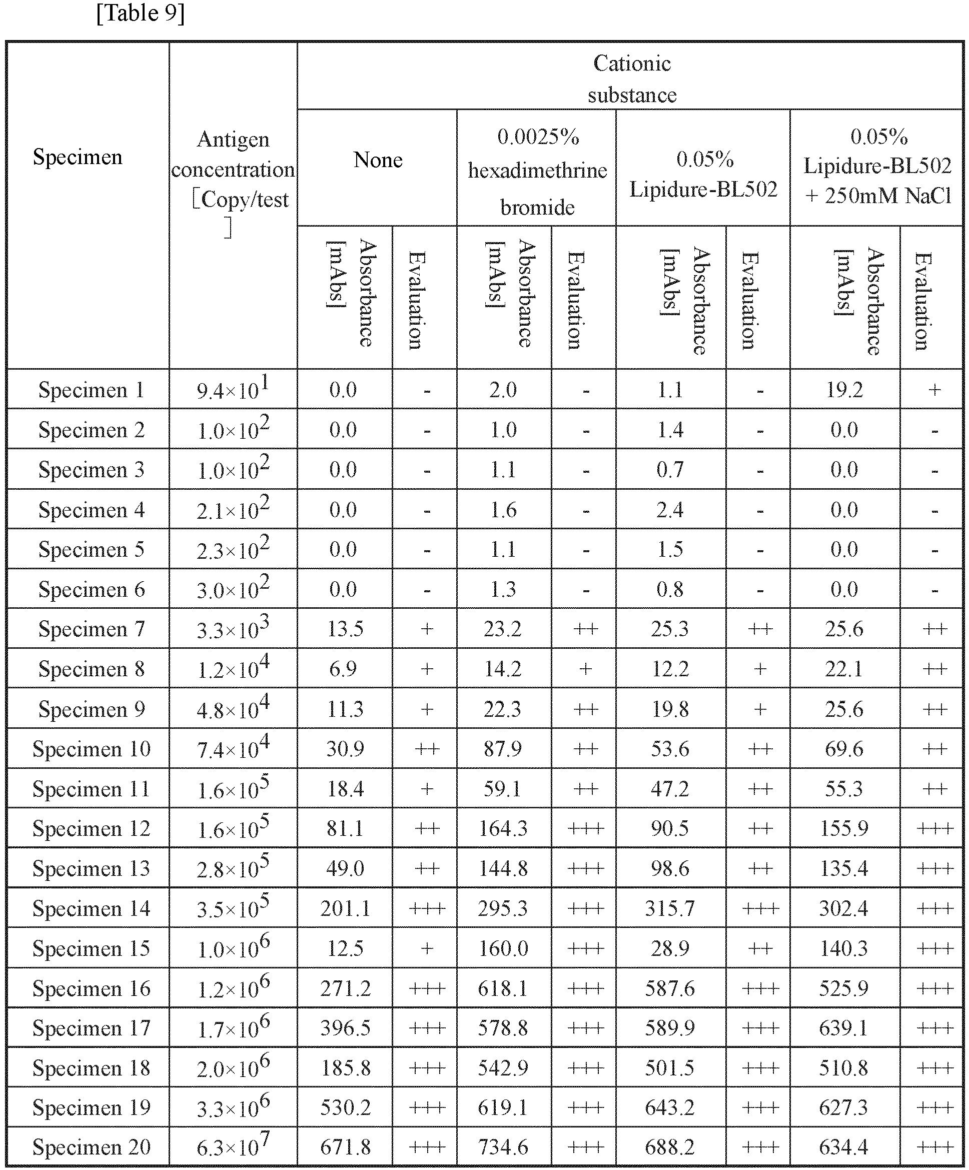

### [Experimental Example 4]

### Evaluation of the effect of cationic substances on specificity

The cationic substances listed in Table 10 were added to the specimen diluent attached to the RapidTesta FLU/NEXT to prepare cation-added specimen diluents. Specimens were extracted from two nasal swabs derived from the same healthy person using 500 µL each of the specimen diluent and the cation-added specimen diluent, and the specimens were filtered with a specimen filtration filter to prepare samples. Measurement by immunochromatography was carried out in the same manner as in Experimental Example 3. The absorbance of the test line was measured 10 minutes and 30 minutes after dropping the sample. The results are shown in Table 10. For all diluents, all specimens were determined to be negative with both 10- and 30-minute measurement times. With respect to the effect of addition of cations to the sample diluent, no adverse effect on the specificity of immunochromatography was observed.

**[Table 10]**

| Specimen | Cationic substance | | | | | |
|---|---|---|---|---|---|---|
| | None | | 0.01% hexadimethrine bromide | | 0.05% Lipidure-BL502 + 250mM NaCl | |
| | 10 min | 30 min | 10 min | 30 min | 10 min | 30 min |
| Specimen 21 | - | - | - | - | - | - |
| Specimen 22 | - | - | - | - | - | - |
| Specimen 23 | - | - | - | - | - | - |
| Specimen 24 | - | - | - | - | - | - |
| Specimen 25 | - | - | - | - | - | - |
| Specimen 26 | - | - | - | - | - | - |
| Specimen 27 | - | - | - | - | - | - |
| Specimen 28 | - | - | - | - | - | - |
| Specimen 29 | - | - | - | - | - | - |
| Specimen 30 | - | - | - | - | - | - |

### [Industrial Applicability]

The present invention can provide an immunoassay method and an immunochromatography kit that can detect proteins with an isoelectric point of 9.5 or higher with high sensitivity. The present invention can also provide a filtration method for a sample containing a protein with an isoelectric point of 9.5 or higher.

### [Reference Signs List]

- 11: Glass
- 11a: Silicon atom
- 11b: Oxygen atom
- 12: Protein with an isoelectric point of 9.5 or higher
- 13: Cationic substance
- 100, 200: Immunochromatography test strip
- 101, 201: Backing sheet
- 102, 202: Conjugate pad
- 103, 203: Absorbent pad
- 104, 204: Insoluble membrane carrier
- 105, 205: Conjugate line
- 106, 206: Test line (detector section)
- 107, 207: Control line
- 208: Sample pad
- 209: Third pad

## Claims

1. An immunoassay method for detecting a protein with an isoelectric point of 9.5 or higher, comprising:
a step of bringing a sample containing the protein with an isoelectric point of 9.5 or higher into contact with a glass material in the presence of a cationic substance.

2. The immunoassay method according to claim 1, wherein the sample is contacted with the cationic substance, followed by bringing the protein with an isoelectric point of 9.5 or higher into contact with the glass material.

3. The immunoassay method according to claim 1 or 2, wherein the glass material is a specimen filtration filter containing glass fibers.

4. The immunoassay method according to claim 1 or 2, which further comprises, prior to the step of bringing the sample into contact with the glass material,
a step of contacting a sample containing the protein with an isoelectric point of 9.5 or more with a specimen diluent,
wherein the cationic substance is contained in the specimen diluent.

5. The immunoassay method according to claim 1 or 2, wherein the cationic substance is at least one selected from the group consisting of a metal salt, a cationic polymer, and a cationic surfactant.

6. The immunoassay method according to claim 1 or 2, wherein the cationic substance is a mixture of a metal salt and a cationic polymer.

7. The immunoassay method according to claim 1 or 2, which is immunochromatography.

8. The immunoassay method according to claim 7, wherein the glass material is at least one selected from the group consisting of a sample pad, a third pad, and a conjugate pad of an immunochromatography test strip.

9. The immunoassay method according to claim 1 or 2, which further comprises:
a step of bringing the protein with an isoelectric point of 9.5 or higher with a first binding partner to which a labeling substance is directly or indirectly bound, thereby forming a first complex;
a step of contacting the first complex with a second binding partner to form a second complex; and
a step of detecting a signal derived from the labeling substance.

10. The immunoassay method according to claim 1 or 2, wherein the protein with an isoelectric point of 9.5 or higher is derived from a respiratory infection virus.

11. The immunoassay method according to claim 10, wherein the respiratory infection virus is a coronavirus.

12. The immunoassay method according to claim 11, wherein the coronavirus is SARS-CoV-2.

13. An immunochromatography kit for detecting a protein with an isoelectric point of 9.5 or higher, comprising a specimen diluent and an immunochromatography test strip, which further comprises:
a cationic substance to be contacted with a sample containing the protein with an isoelectric point of 9.5 or higher; and
a glass material to be contacted with the sample containing the protein having with an isoelectric point of 9.5 or higher.

14. The immunochromatography kit according to claim 13, which has a specimen filtration filter, wherein the specimen filtration filter comprises the glass material.

15. The immunochromatography kit according to claim 13, which comprises at least one member selected from the group consisting of a sample pad in the immunochromatography test strip, a third pad in the immunochromatography test strip, and a conjugate pad in the immunochromatography test strip, wherein the at least one member comprises the glass material.

16. The immunochromatography kit according to any one of claims 13 to 15, wherein the cationic substance is contained in the specimen diluent.

17. The immunochromatography kit according to any one of claims 13 to 15, wherein the cationic substance is at least one selected from the group consisting of a metal salt, a cationic polymer, and a cationic surfactant.

18. The immunochromatography kit according to any one of claims 13 to 15, wherein the cationic substance is a mixture of a metal salt and a cationic polymer.

19. The immunochromatography kit according to any one of claims 13 to 15, wherein the protein with an isoelectric point of 9.5 or higher is derived from a respiratory infection virus.

20. The immunochromatography kit according to claim 19, wherein the respiratory infection virus is a coronavirus.

21. The immunochromatography kit according to claim 20, wherein the coronavirus is SARS-CoV-2.

22. A specimen diluent containing a cationic substance for use in the immunoassay method of claim 1 or 2.

23. A filtration method for a sample containing a protein with an isoelectric point of 9.5 or higher, the method comprising:
a step of contacting a sample containing the protein with an isoelectric point of 9.5 or more with a specimen diluent, and
a step of filtering the sample diluted with the specimen diluent using a specimen filtration filter containing a glass material,
wherein the specimen diluent comprises a cationic substance.

24. An adsorption suppression method for suppressing adsorption of a protein with an isoelectric point of 9.5 or higher to a glass material in an immunoassay method for detecting a protein with an isoelectric point of 9.5 or higher, the method comprising:
a step of bringing a sample containing the protein with an isoelectric point of 9.5 or higher into contact with a glass material in the presence of a cationic substance.
